# EUROPÄISCHE PATENTSCHRIFT

(11) **EP 1 226 833 B1**
(45) Veröffentlichungstag und Bekanntmachung des Hinweises auf die Patenterteilung: **11.05.2005**
(21) Anmeldenummer: 01130768.3
(22) Anmeldetag: 22.12.2001
(51) Int. Cl.: A61L 2/00, A61L 2/14, A61L 2/18, A61L 2/20, B65B 55/02

(54) **Hydrogenperoxid-Plasma-Sterilisationsverfahren für die schonende Sterilisation temperaturempfindlicher Produkte**
Hydrogen peroxide gas plasma sterilization process for gentle sterilization of temperature sensitive products
Procédé de stérilisation par plasma de péroxyde d'hydrogène pour la stérilisation douce des produits thermo-sensibles

(30) Priorität: 26.01.2001 DE 10103706
(43) Veröffentlichungstag der Anmeldung: 31.07.2002
(73) Patentinhaber: ZLB Behring GmbH, 35041 Marburg (DE)
(72) Erfinder: Metzner, Hubert, 35041 Marburg (DE); Lemmer, Joerg, 35085 Ebsdorfergrund (DE); Naumann, Horst, 35037 Marburg (DE)

(56) Entgegenhaltungen:
- EP-A- 0 679 407
- EP-A- 0 799 621
- EP-A- 1 040 839
- US-A- 4 643 876
- US-A- 6 159 422

## Beschreibung

Gegenstand der Erfindung ist ein Verfahren zur Hydrogenperoxid-Plasma-Sterilisation, wobei die Einstellung der Kammertemperatur über den gesamten Verlauf bei weniger als 39°C liegt, und Behälter mit temperaturempfindlichen Produkten effektiv sterilisiert werden können, ohne dass die temperaturempfindlichen Produkte einen signifikanten Aktivitätsabfall bzw. Abbau zeigen.

Die äußerliche Sterilisation von pharmazeutischen Behältern, die Produkte enthalten, die empfindlich gegenüber Temperatureinflüssen oder Bestrahlung sind, ist ein generelles Problem, das bislang nicht befriedigend gelöst ist.

Die Autoklavierung scheidet für biologische Produkte praktisch immer aus, da dieser thermischen Belastung auch die stabilsten Produkte in der Regel nicht standhalten.

Die industriell genutzte Sterilisation mittels Ethylenoxid benötigt regelmäßig Temperaturen von ca. 40-50°C, typischerweise über einen Zeitraum von insgesamt ca. 24-48 Stunden, u.a. auch um das restliche Ethylenoxid möglichst vollständig zu entfernen. Aber auch diese Bedingungen sind für temperaturempfindliche Produkte oft nicht akzeptabel. Hinzu kommt, dass mögliche Restmengen an Ethylenoxid oder dessen Reaktionsprodukten in der Verpackung aufgrund dessen Kanzerogenität bzw. Toxizität ein Nachteil dieser Methode darstellen.

Ein anderes Verfahren, die Bestrahlung mit γ-Strahlen oder Elektronenstrahlen ist auch für empfindliche Produkte, besonders im flüssigen Zustand, in der Regel nicht geeignet, da es z.B. mit Aktivitätsverlusten und/oder Produktdegradation verbunden ist.

In den achtziger Jahren wurde ein Sterilisationsverfahren beschrieben (EP-A 0 302 420), bei dem unter reduziertem Druck (< 1 Torr) mit Wasserstoffperoxid in der Gasphase eine Sterilisation angestrebt wurde. Obwohl dieser Prozess auch bei Raumtemperatur beschrieben wurde, ist seine Sterilisationseffizienz nicht ausreichend, um daraus ein sicheres Verfahren für die Routineanwendung zu entwickeln. Erst eine Temperaturerhöhung auf zumindest 40°C erhöht die Effizienz dieses Verfahrens.

Auch bei dem Sterilisationsverfahren mittels Wasserstoffperoxid und Zündung eines Gasplasmas sind Kammertemperaturen von 45°C bzw. 40-45°C üblich (T.C.V. Penna, C.A.M. Ferraz, and M.A. Cassola; Infection Control and Hospital Epidemiology 20: 465-472 (1999) und R.F. Morrissey; Biomedical Instrumentation & Technology 30: 404-406 (1996)), lt. US-Patentschrift 4.643,876 bzw. EP 207 417 werden sogar Temperaturen von 57°C im zu sterilisierenden Material gemessen, da das Verfahren selbst zu einer Erwärmung des Sterilisationsgutes führt. Obwohl dieses Verfahren als Niedertemperaturverfahren bzw. Niedertemperaturplasma beschrieben wurde, sind die verwendeten bzw. erreichten Temperaturen immer noch so hoch, dass empfindliche Produkte bei dieser Temperaturbelastung zumindest teilweise geschädigt werden.

Das Hydrogenperoxid-Plasma-Sterilisationsverfahren wird unter der Bezeichnung STERRAD®-Verfahren seit Anfang der neunziger Jahre in Europa und USA im wesentlichen zur Sterilisation medizinischer Bestecke eingesetzt. So ist auch die große Mehrzahl der entsprechenden Geräte im Krankenhausbereich zu finden. Allerdings gibt es auch einige wenige Anwendungen z.B. bei medizinisch einsetzbaren Produkten, Gerätschaften oder Einmalartikeln, bei denen sich die Verwendung von Ethylenoxid oder γ-Bestrahlung aus Kompatibilitätsgründen verbot. Diese Produkte können dann oft bei den dem Hydrogenperoxid-Plasma-Sterilisationsverfahren eigenen Temperaturen von ca. 45°C Kammertemperatur ohne Verlust der Funktionalität sterilisiert werden.

Das STERRAD®-Verfahren ist bislang auf eine Kammertemperatur von 45°C (STERRAD® 100) oder > 39°C (STERRAD® GMP 100) beschränkt, da man nach den bekannten Ergebnissen (z.B. EP-A 0 302 420) davon ausgehen musste, daß eine effektive Sterilisierung erst bei Temperaturen von mehr als 39 °C erreicht werden kann. Eigene Versuche mit dem Hydrogenperoxid-Plasma-Sterilisationsverfahren bei 45°C Standard-Kammertemperatur mit biologischen Produkten unter den als schonend angenommenen Bedingungen nach dem Stand der Technik zeigten, daß empfindliche biologische Materialien zum Teil deutliche oder vollständige Aktivitätseinbußen erlitten (siehe Beispiel 1). Diese können somit nicht unter den bekannten Bedingungen des STERRAD®-Verfahrens sterilisiert werden.

Der vorliegenden Erfindung lag nun die Aufgabe zugrunde, eine Verfahrensweise zu entwickeln, die es erlaubt, empfindliche, biologische bzw. therapeutische Produkte im festen oder im flüssigen Zustand in ihrem Endbehälter (Primärpackmittel) äußerlich zu sterilisieren. Dabei sollte die Auswahl des Endbehälters gewährleisten, dass eine Beeinträchtigung des Produktes durch das Verfahren nicht erfolgt. Ferner sollte eine Sterilisation des Produktes in zwei Umverpackungen (Sekundärpackmittel) möglich sein.

Diese Aufgabe wurde dadurch gelöst, dass am Beispiel der temperaturempfindlichen Komponenten eines Fibrinklebers eine. Modifikation des Hydrogenperoxid-Plasma-Sterilisationsverfahrens bei weiter reduzierter Temperatur entwickelt werden konnte, die es erlaubt, schnell und schonend Endbehälter mit empfindlichen Produkten auch in Umverpackungen äußerlich effektiv zu sterilisieren.

Es wurde nun gefunden, dass Behälter mit temperaturempfindlichen Produkten unter modifizierten Bedingungen mit Hydrogenperoxid / Plasma wirksam sterilisiert werden können, ohne dass dabei die bislang üblichen Temperaturen angewendet werden müssen bzw. auftreten. Bei Kammertemperaturen unterhalb 39°C können sowohl Produktstabilität als auch Sterilität errreicht werden. Bei Kammertemperaturen um 20-39°C, besonders auch bei ca. 25-35°C, lassen sich die Sterilisationen sehr produktschonend durchführen. Durch Einführung dieser Niedrigtemperatur-Modifikation können somit Endbehälter mit empfindlichen Produkten wie z.B. Proteinen, Peptiden etc. in Lösung effektiv sterilisiert werden. Maßgeblich für die niedrige Produkttemperatur ist dabei sowohl die geringe Kammertemperatur als auch die Vermeidung eines zu hohen Energieeintrages z.B. bei der Injektion des Wasserstoffperoxid sowie bei der Plasmabildung.

Vor Durchführung der Sterilisation können die Produkte ggfs. einem Vorplasma ausgesetzt werden, um Feuchtigkeit zu entfernen wie in EP 707186 beschrieben oder um die Produkttemperatur der Kammertemperatur weiter anzupassen.

Bei dem anzuwendenden Sterilisationszyklus können die sog. Injektionsphase(n) und die Diffusionsphase(n) (ggfs. mit gleichzeitiger Belüftung) in ihrer Zeitdauer variiert werden, bevorzugt zwischen ca. 1 und 60 Minuten. Auf die Belüftung während der Diffusionsphase kann ggfs. auch verzichtet werden, besonders dann, wenn das zu sterilisierende Produkt eine einfache Geometrie aufweist.

Die Injektion von Wasserstoffperoxidlösung kann -besonders bei voller Kammerbeladung- auch ein- oder mehrfach wiederholt werden, um einen ausreichenden Wasserstoffperoxidgehalt in der Gasphase und so einen erhöhten Abtötungsgrad zu erzielen. Falls notwendig kann auch der gesamte Zyklus oder Teile davon ein- oder mehrfach wiederholt werden, wobei allerdings entsprechend dem beanspruchten Verfahren eine Einstellung der Kammertemperatur von < 39°C zu beachten ist, um die Erwärmung zu begrenzen.

Wenn die Diffusionsphase mit Belüftung entfällt, kann auf die Injektionsphase, die Wiederherstellung eines ausreichenden Vakuums und das Plasma wieder ein Halbzyklus aus Injektion, Vakuum und Plasma folgen. Dies kann bei einfachen Produktgeometrien die Zyklusdauer reduzieren.

Durch diese Methodik können die Endbehälter von empfindlichen biologischen Produkten wie z.B. Proteinen oder Peptiden in kurzen Zyklen bei niedriger Temperatur sterilisiert werden. Wie im Falle von Blutplasmaproteinen gezeigt werden konnte, ist mit dem erfindungsgemäßen Verfahren eine produktschonende Sterilisation der Endbehälter möglich. Solche Produkte können dann überall dort eingesetzt werden, wo eine sterile Handhabung notwendig ist.

Es ist aber auch prinzipiell möglich, die beschriebene Verfahrensweise für andere, empfindliche biologische Produkte wie DNA, RNA, Lipide, zelluläre Produkte etc. anzuwenden. Durch die kurze und geringe Temperaturbelastung sind bei den genannten Produkten keine signifikanten Verluste zu erwarten.

Das erfindungsgemäße Verfahren kann aber auch generell für die Sterilisation von Endbehältern eingesetzt werden, enthaltend temperaturempfindliche, nichtbiologische Produkte. Dies können z.B. synthetische Verbindungen oder therapeutisch einsetzbare Produkte sein, die aufgrund ihrer Temperatursensitivität bei den üblichen Verfahren teilweise oder vollständig inaktiviert oder geschädigt werden.

Bei der Auswahl der Endbehälter, d.h. der Primärpackmittel, ist zu beachten, dass bewegliche Verschlüsse wie Stopfen, Kolbenstopfen oder Kappen so fixiert werden, dass im Vakuum kein Öffnen und keine Undichtigkeit der Primärpackmittel auftritt. Dies kann z.B. durch entsprechende Vorrichtungen verhindert werden, sei es, dass die Verschlüsse direkt fixiert werden oder sei es, dass durch eine entsprechende Sekundärverpackung Sorge getragen wird, dass keine Undichtigkeit bzw. Stopfenoder Kolbenstopfenverschiebung auftreten kann (siehe Zeichnung).

Ein typischer Halbzyklus des erfindungsgemäßen Verfahrens bei reduzierter Temperatur besteht aus den folgenden Elementen:

### Vorbereitung (Bei Bedarf durchzuführen):

- Absenkung des Druckes in der Behandlungskammer, bevorzugterweise auf etwa 100 bis 800 mTorr, ganz bevorzugterweise auf etwa 300 bis 600 mTorr
- Anlegen eines Vorplasmas, bevorzugterweise für etwa 1 bis 30 min, ganz bevorzugterweise für etwa 2 bis 15 min,
- Belüftung, bevorzugterweise in weniger als 5 min, ganz bevozugterweise in weniger als 1 min.

### Durchführung eines Halbzyklus bei einer eingestellten Kammertemperatur von < 39°C, bevorzugterweise bei etwa 20-35 °C:

- Absenkung des Druckes in der Behandlungskammer, bevorzugterweise auf etwa 100 bis 800 mTorr, ganz bevorzugterweise auf etwa 300 bis 600 mTorr
- Einbringen des Wasserstoffperoxids, in der Regel durch ein- oder mehrmaliges direktes Verdampfen einer Lösung im Vakuum mit anschließender Verteilung in der Kammer (Injektion), bevorzugterweise innerhalb 20 min, ganz bevorzugterweise innerhalb 15 min,
- Ggfs. zusätzliche Diffusionsphase des Wasserstoffperoxids (ggfs. mit Belüftung der Kammer), je nach Anforderungen des zu sterilisierenden Produktes, bevorzugterweise für 1 bis 30 min, ganz bevorzugterweise für 3 bis 15 min,
- Erneutes Absenkung des Druckes und Wiederherstellung eines ausreichenden Vakuums
- Erzeugung eines Plasmas, bevorzugterweise für 0,5 bis 10 min, ganz bevorzugterweise für 1 bis 5 min,
- Belüftung

Ein gesamter Sterilisationszyklus umfasst meist zwei Halbzyklen. Diese Unterteilung wurde aus Gründen der Verfahrensvalidierung getroffen. Wird in einem solchen Halbzyklus ein Abreicherungsfaktor mit Modell-Mikroorganismen, wie z.B. den Sporen des Bac. stearothermophilus, von ≥6 log₁₀ KBE (Kolonie bildende Einheiten) erzielt, kann mit ausreichender Sicherheit von einem effektiven Verfahren gesprochen werden.

Der Gegenstand der Erfindung ist im wesentlichen in den Ansprüchen beschrieben. Es wird durch die Beispiele 2-6 gezeigt, wie anhand eines modifizierten Sterilisationsverfahrens von Primärpackmitteln, enthaltend temperaturempfindliche Proteine wie z.B. die Komponenten eines Fibrinklebers, eine wirksame Sterilisation bei niedriger Temperatur möglich ist ohne die Eigenschaften der Proteinkomponenten zu beeinträchtigen.

Beispiel 1 zeigt das Ergebnis eines Hydrogenperoxid-Plasma-Sterilisationslaufes unter Standard-Temperaturbedingungen nach dem Stand der Technik. Die folgenden Beispiele (2-6) sollen das Prinzip des modifizierten Verfahrens bei niedriger Temperatur veranschaulichen:

### Vergleichsbeispiel 1

Verschiedene Proteinlösungen (wesentlicher Bestandteil von Proteinlösung 1: Fibrinogen, von Proteinlösung 2: Faktor XIII und von Proteinlösung 3: Thrombin) wurden in Glaskarpulen abgefüllt und in Beuteln bestehend aus Tyvek- und einer durchsichtigen Kunststofffolie eingeschweißt. Anschließend wurden die Beutel in Körbe geschichtet und in einem STERRAD® GMP 100 Sterilisator (Lieferant: Johnson & Johnson Medical GmbH, 22844 Norderstedt, Deutschland) mit einem Hydrogenperoxid-Plasma-Sterilisationsverfahren entsprechend der folgenden Parameter behandelt. Für die Durchführbarkeit der Beispiele ist die exakte Zusammensetzung der verwendeten Proteinlösungen unwesentlich. Es handelt sich um dem Fachmann ansich bekannte Lösungen von therapeutisch eingesetzten biologischen Proteinen, natürlichen oder rekombinanten Ursprungs.

### Vorbereitung:

Einstellung der Kammertemperatur auf 45°C. Beladung der Kammer: Korb beschickt mit Produkt-haltigen Primärpackmitteln in Beuteln.

### Durchführung des 1. Halbzyklus:

- Vakuum:: auf ca. 400 mTorr
- Injektion:: ca. 6 min (1800 µl 59% H₂O₂)
- Diffusion:: 10 min (inkl. Belüftung)
- Vakuum:: auf ca. 400-500 mTorr
- Plasma:: 2 min

Durchführung eines 2., 3. und 4. Halbzyklus (entsprechend 1. Halbzyklus) direkt im Anschluß an Halbzyklus 1.

Ergebnisse der Sterilisationsläufe entsprechend Beispiel 1: Stabilität der untersuchten Proteinlösungen:

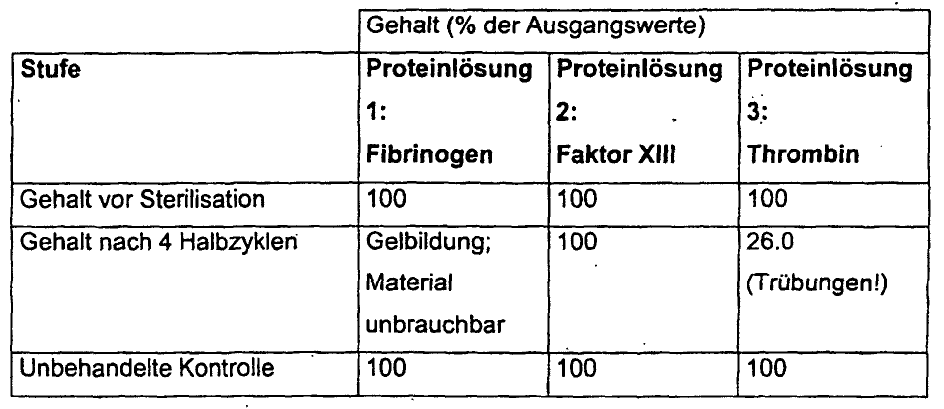

Wie aus der Tabelle zu Beispiel 1 ersichtlich ist, tritt im Falle der Proteinlösung 1 (enthaltend Fibrinogen) sowie der Proteinlösung 3 (enthaltend Thrombin) eine temperaturbedingte Aggregation oder Abbau bzw. Denaturierung auf. D.h. beide Produkte sind durch das Hydrogenperoxid-Plasma-Sterilisationsverfahren entsprechend dem Stand der Technik für den vorgesehenen Einsatz unbrauchbar geworden.

### Beispiel 2

Verschiedene Proteinlösungen (wesentlicher Bestandteil von Proteinlösung 1: Fibrinogen, von Proteinlösung 2: Faktor XIII und von Proteinlösung 3: Thrombin) wurden in Glaskarpulen abgefüllt und in Beuteln bestehend aus Tyvek- und einer durchsichtigen Kunststofffolie eingeschweißt. Anschließend wurden die Beutel in Körbe geschichtet und in einem STERRAD® GMP 100 Sterilisator mit einem Hydrogenperoxid-Plasma-Sterilisationsverfahren entsprechend folgenden Parametern behandelt.

### Vorbereitung:

Einstellung der Kammertemperatur auf: 30°C

Beladung der Kammer: unterer Korb dicht gepackt mit den beschriebenen Beuteln enthaltend wassergefüllte Primärpackmittel; oberer Korb beschickt mit Produkt-haltigen Beuteln sowie zusätzlich einem Temperatursensor.

### Nach erfolgter Beladung Durchführung eines "Vorplasmas":

- Vakuum:: auf ca. 400 mTorr
- Vorplasma:: 5 min
Belüftung (kurz)

### Durchführung eines 1. Halbzyklus:

- Vakuum:: auf ca. 400 mTorr
- Injektion:.: ca. 12 min (1800 µl 59% H₂O₂)
- Diffusion:: 5 min (inkl. Belüftung)
- Vakuum:: auf ca. 400-500 mTorr
- Plasma:: ca. 2 min

Vakuum (kurz) und Belüftung (zur Probenentnahme) Durchführung eines 2. Halbzyklus (entsprechend 1. Halbzyklus) direkt im Anschluß an Halbzyklus 1.

Ergebnisse der Sterilisationsläufe entsprechend Beispiel 2: Stabilität der untersuchten Proteinlösungen:

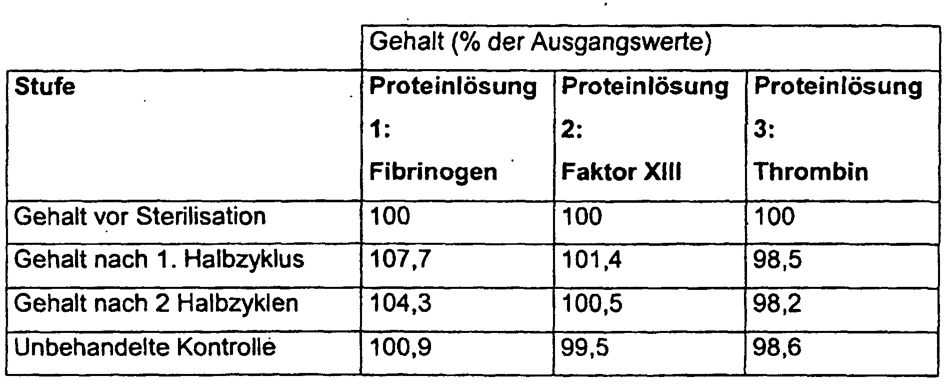

### Beispiel 3

Verschiedene Proteinlösungen (wesentlicher Bestandteil von Proteinlösung 1: Fibrinogen, von Proteinlösung 2: Faktor XIII und von Proteinlösung 3: Thrombin) wurden in Glaskarpulen abgefüllt und in Beuteln bestehend aus Tyvek- und einer durchsichtigen Kunststofffolie eingeschweißt. Anschließend wurden die Beutel in Körbe geschichtet und in einem STERRAD® GMP 100 Sterilisator mit einem Hydrogenperoxid-Plasma-Steritisationsverfahren entsprechend folgenden Parametern behandelt.

### Vorbereitung:

Einstellung der Kammertemperatur auf 30°C.

Beladung der Kammer: unterer Korb dicht gepackt mit den beschriebenen Beuteln enthaltend wassergefüllte Primärpackmittel; oberer Korb beschickt mit Produkt-haltigen Beuteln sowie zusätzlich einem Temperatursensor.

### Nach erfolgter Beladung Durchführung eines "Vorplasmas":

- Vakuum:: auf ca. 400-500 mTorr
- Vorplasma:: 5 min

Belüftung (kurz)

Durchführung eines 1. Halbzyklus:
- Vakuum: .: auf ca. 400 mTorr
- Injektion:: ca. 17 min (1800 µl 59% H₂O₂)
- Vakuum:: auf ca. 400-500 mTorr
- Plasma:: 2 min

Vakuum (kurz) und Belüftung (zur Probenentnahme)

Durchführung eines 2. Halbzyklus (entsprechend 1. Halbzyklus) direkt im Anschluß an Halbzyklus 1.

Ergebnisse der Sterilisationsläufe entsprechend Beispiel 3: Stabilität der untersuchten Proteinlösungen: .

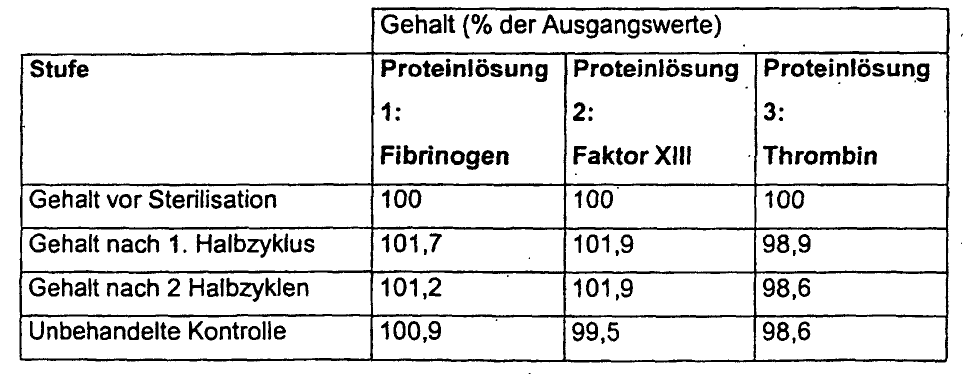

### Beispiel 4

Verschiedene Proteinlösungen (wesentlicher Bestandteil von Proteinlösung 1: Fibrinogen, von Proteinlösung 2: Faktor XIII und von Proteinlösung 3: Thrombin) wurden in Glaskarpulen abgefüllt und in Beuteln bestehend aus Tyvek- und einer durchsichtigen Kunststofffolie eingeschweißt. Anschließend wurden die Beutel in. Körbe geschichtet und in einem STERRAD® GMP 100 Sterilisator mit einem Hydrogenperoxid-Plasma-Sterilisationsverfahren entsprechend den folgenden Parametern behandelt. Neben Produkt-befüllten Primärpackmitteln wurden auch Karpulen und Sporenstreifen eines Testorganismus (Bac. stearothermophilus) doppelt in Tyvek-Beuteln eingeschweißt, um die Sterilisationseffektivität zu überprüfen.

### Vorbereitung:

Einstellung der Kammertemperatur auf: 35°C. Beladung der Kammer: unterer Korb dicht gepackt mit den beschriebenen Beuteln enthaltend wassergefüllte Primärpackmittel; oberer Korb beschickt mit Produkt-haltigen Beuteln, Beuteln enthaltend Sporenstreifen und Primärpackmittel sowie zusätzlich einem Temperatursensor.
Nach erfolgter Beladung Durchführung eines "Vorplasmas"
- Vakuum:: auf ca. 400-500 mTorr
- Vorplasma:: 5 min
Belüftung (kurz)

### Durchführung eines 1. Halbzyklus:

- Vakuum:: auf ca. 400 mTorr
- Injektion:: ca. 12 min (1800 µl 59% H₂O₂)
- Diffusion:: 5 min (inkl. Belüftung)

- Vakuum:: auf ca. 400-500 mTorr
- Plasma:: 2 min

Vakuum (kurz) und Belüftung (zur Probenentnahme)

Durchführung eines 2. Halbzyklus (entsprechend 1. Halbzyklus) direkt im Anschluß an Halbzyklus 1.

Ergebnisse der Sterilisationsläufe entsprechend Beispiel 4: Stabilität der untersuchten Proteinlösungen:

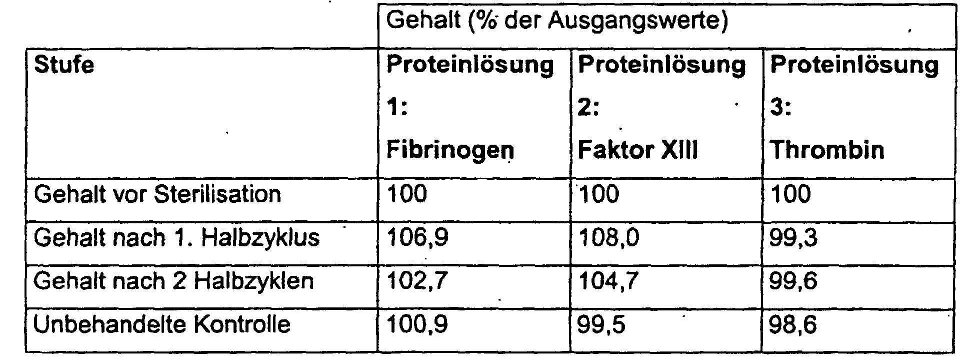

Sterilitätsauswertung von 8 Sporenstreifen behandelt in einem Halbzyklus:

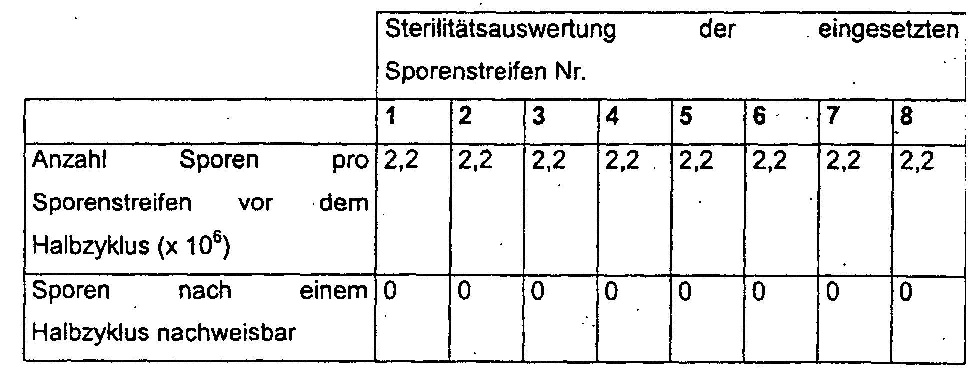

### Beispiel 5

Verschiedene Proteinlösungen (wesentlicher Bestandteil von Proteinlösung 1: Fibrinogen, von Proteinlösung 2: Faktor XIII und von Proteinlösung 3: Thrombin) wurden in Glaskarpulen abgefüllt und in Beuteln bestehend aus Tyvek- und einer durchsichtigen Kunststofffolie eingeschweißt. Anschließend wurden die Beutel in Körbe geschichtet und in einem STERRAD® GMP 100 Sterilisator mit einem Hydrogenperoxid-Plasma-Sterilisationsverfahren entsprechend folgenden Parametern behandelt.

### Vorbereitung:

Einstellung der Kammertemperatur auf: 35°C
Beladung der Kammer: unterer Korb dicht gepackt mit den beschriebenen Beuteln enthaltend wassergefüllte Primärpackmittel; oberer Korb beschickt mit Produkt-haltigen Beuteln sowie zusätzlich einem Temperatursensor.

### Nach erfolgter Beladung Durchführung eines "Vorplasmas":

- Vakuum:: auf ca. 400-500 mTorr
- Vorplasma:: 5 min

Belüftung (kurz)

### Durchführung eines 1. Halbzyklus:

- Vakuum:: auf ca. 400 mTorr
- 1. Injektion:: ca. 2 min (1800 µl 59% H₂O₂)
- 2. Injektion:: ca. 10 min (1800 µl 59% H₂O₂)
- Diffusion:: 5 min (inkl. Belüftung)
- Vakuum:: auf ca. 400-500 mTorr
- Plasma:: 2 min

Vakuum (kurz) und Beginn mit nächstem Halbzyklus oder Belüftung (zur Probenentnahme)

Durchführung eines 2., 3. und 4. Halbzyklus (entsprechend 1. Halbzyklus) direkt im Anschluß an Halbzyklus 1.

Ergebnisse der Sterilisationsläufe entsprechend Beispiel 5: Stabilität der untersuchten Proteinlösungen:

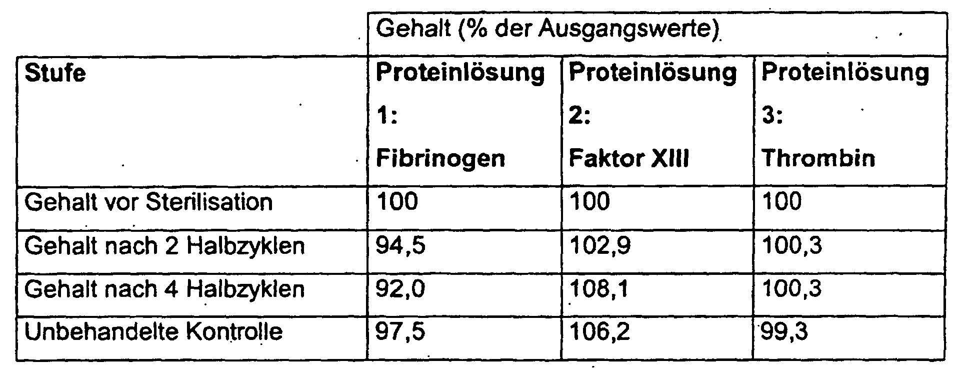

### Beispiel 6

Zur Prüfung der Sterilisationseffektivität des Verfahrens in einer maximal beladenen Kammer mit kommerziellen Verpackungsmaterialien wurden Wasser-gefüllte Glaskarpulen in Umverpackungen bestehend aus PET Hartblistern, verschlossen mit Tyvek-Papier sowie zusätzlich mit Beuteln, bestehend aus Tyvek-Papier und einer durchsichtigen Kunststofffolie, eingeschweißt. Anschließend wurden die Packungen dicht in Körbe geschichtet und in einem STERRAD® GMP 100 Sterilisator mit einem Hydrogenperoxid-Plasma-Sterilisationsverfahren entsprechend den folgenden Parametern behandelt.

.Bei acht Blisterpackungen, verteilt an unterschiedlichen Positionen der Körbe, wurden Sporenstreifen eines Testorganismus (Bac. stearothermophilus) an die schlechtest zugänglichen Positionen in die doppelte Verpackung eingebracht und zugeschweißt, um die Sterilisationseffektivität zu überprüfen.

### Vorbereitung:

Einstellung der Kammertemperatur auf: 32°C.
Beladung der Kammer: unterer und oberer Korb dicht gepackt mit den beschriebenen Verpackungen (172 Stück) enthaltend wassergefüllte Primärpackmittel; 8 Verpackungen enthielten neben den Primärpackmitteln auch Sporenstreifen (zwischen Karpule und Hartblister bzw. zwischen Karpulenende und Stopfenhalterung) und wurden nach einem bestimmten Plan in den Körben verteilt.

### Durchführung eines "Vorplasmas":

- Vakuum:: auf ca. 400-500 mTorr
- Vorplasma:: 5 min

### Durchführung eines Halbzyklus:

- Vakuum:: auf ca. 400-500 mTorr
- 3 Injektionen:: Dauer ca. 3, 6 bzw. 6 min (je 1800 µl 59% H₂O₂)
- Diffusion:: 5 min (inkl. Belüftung)
- Vakuum:: auf ca. 400-500 mTorr
- Plasma:: 2 min

Vakuum (kurz) und Belüftung (zur Probenentnahme)

Ergebnis des Sterilisationslaufes entsprechend Beispiel 6: Sterilitätsauswertung von 8 Sporenstreifen behandelt in einem Halbzyklus:

Von den eingesetzten Sporenstreifen wurden alle vollständig inaktiviert, auch diejenigen an den schlechtest zugänglichen Positionen, d.h. dieses Verfahren ermöglichte eine wirksame Sterilisation von mehr als 10⁶ Sporen in einem Halbzyklus.

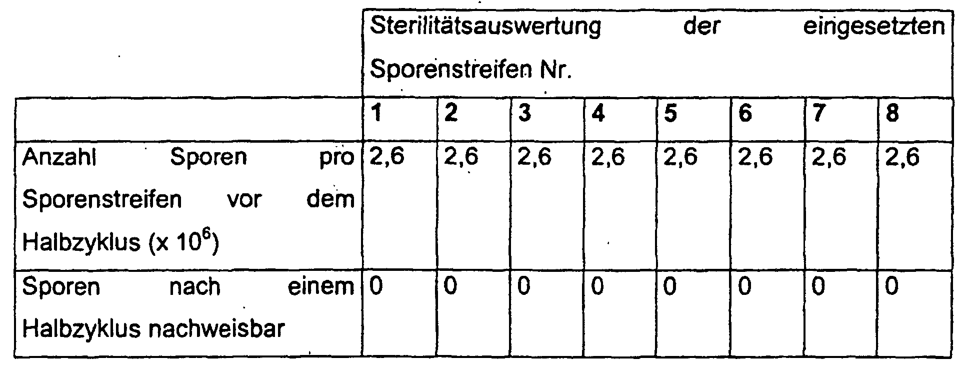

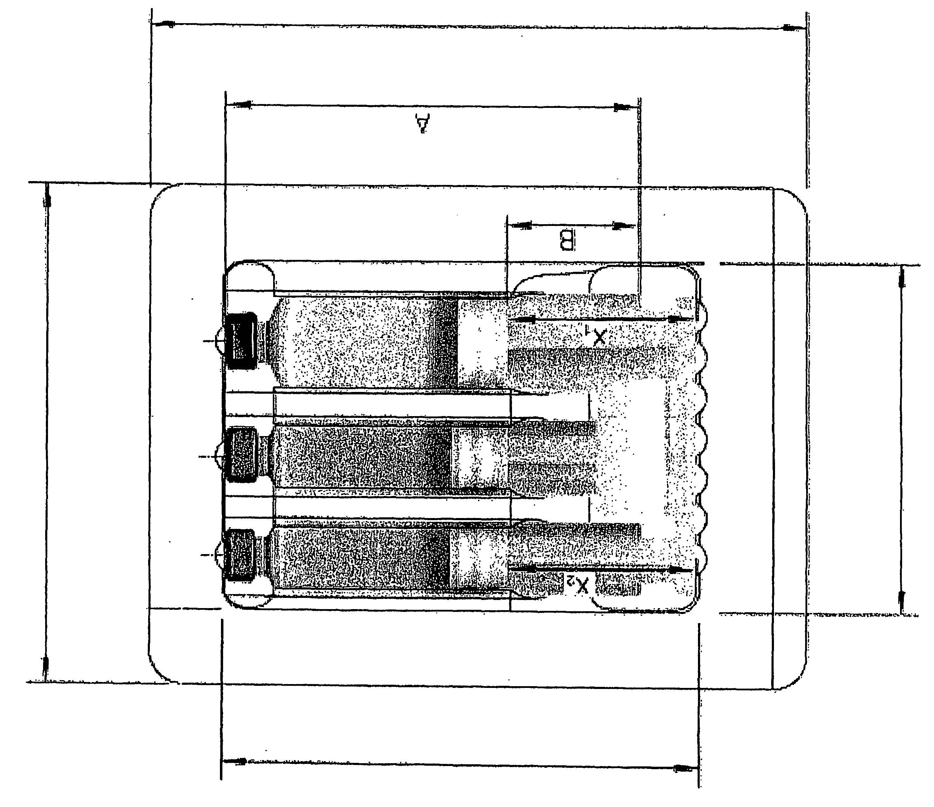

## Patentansprüche

1. Verfahren zur Hydrogenperoxid-Plasma-Sterilisation mittels Injektion einer wässrigen Wasserstoffperoxidlösung in eine Behandlungskammer, **dadurch gekennzeichnet, dass**
a) das zu sterilisierende Produkt nicht mehr als einem Vorplasma ausgesetzt wird,
b) die Kammertemperatur über den gesamten Verlauf weniger als 39°C beträgt,
c) ein Keimabreicherungsfaktor von größer als 6 log10 Kolonie bildende Einheiten (KBE) in einem Durchgang erzielt wird, und
d) Behälter mit temperaturempfindlichen Produkten effektiv sterilisiert werden können, ohne dass die temperaturempfindlichen Produkte einen signifikanten Aktivitätsabfall bzw. Abbau zeigen

2. Hydrogenperoxid-Plasma-Sterilisationsverfahren nach Anspruch 1, **dadurch gekennzeichnet, dass** die Produkttemperatur beim Sterilisationsvorgang nicht über 40°C ansteigt.

3. Hydrogenperoxid-Plasma-Sterilisationsverfahren nach mindestens einem der Ansprüche 1-2, **dadurch gekennzeichnet, dass** es sich bei den temperaturempfindlichen Produkten um temperaturempfindliche biologische Materialien handelt.

4. Hydrogenperoxid-Plasma-Sterilisationsverfahren nach mindestens einem der Ansprüche 1-3, wobei folgende Schritte enthalten sind:
- Einbringen eines zu sterilisierenden Behälters, enthaltend ein temperaturempfindliches Material, in eine Behandlungskammer,
- Absenken des Druckes in der Behandlungskammer
- Einbringen des Wasserstoffperoxids,
- gegebenenfalls zusätzliche Diffusionsphase des Wasserstoffperoxids, gegebenenfalls mit Belüftung,
- Erneutes Absenkung des Druckes und Wiederherstellung eines ausreichenden Vakuums
- Erzeugung eines Plasmas,
- Belüftung

5. Hydrogenperoxid-Plasma-Sterilisationsverfahren nach mindestens einem der Ansprüche 1-4, **dadurch gekennzeichnet, dass** es sich bei den temperaturempfindlichen Produkten um Proteine, Peptide, Nucleinsäuren, Lipide, oder zellhaltige Materialien handelt.

6. Verfahren nach mindestens einem der Ansprüche 1-4, **dadurch gekennzeichnet, dass** es sich bei dem temperaturempfindlichen Produkt um eine Fibrinogen-haltige Lösung handelt, und dass der Gehalt an gerinnbarem Fibrinogen nicht mehr als 8% abfällt und dass das Fibrinogen keine signifikante Viskositätszunahme oder Gelbildung zeigt.

7. Verfahren nach mindestens einem der Ansprüche 1-4, **dadurch gekennzeichnet, dass** es sich bei dem temperaturempfindlichen Produkt um eine Faktor XIII-haltige Lösung handelt.

8. Verfahren nach mindestens einem der Ansprüche 1-4, **dadurch gekennzeichnet, , dass** es sich bei dem temperaturempfindlichen Produkt um eine Thrombin-haltige Lösung handelt.

9. Verfahren nach mindestens einem der Ansprüche 1-4, **dadurch gekennzeichnet, daß** es sich bei dem temperaturempfindlichen Produkt um die Komponenten eines Gewebeklebers handelt.

10. Verfahren nach mindestens einem der Ansprüche 1-4, **dadurch gekennzeichnet, daß** es sich bei dem temperaturempfindlichen Produkt um die Komponenten eines Fibrinklebers handelt.

11. Verfahren nach den Ansprüchen 1-10, **dadurch gekennzeichnet, dass** die Behälter einmal mit Materialien umhüllt sind, die mindestens teilweise durchlässig für Wasserstoffperoxid sind.

12. Verfahren nach den Ansprüchen 1-10, **dadurch gekennzeichnet, dass** die Behälter mehrfach mit Materialien umhüllt sind, die mindestens teilweise durchlässig für Wasserstoffperoxid sind.

## Claims

1. A method for hydrogen peroxide plasma sterilization by means of injecting an aqueous hydrogen peroxide solution into a treatment chamber, wherein
a) the product to be sterilized is exposed to no more than a preplasma,
b) the chamber temperature is less than 39°C throughout,
c) a microbe reduction factor of greater than 6 log10 colony forming units (CFU) is achieved in one run, and
d) containers with temperature-sensitive products can be efficiently sterilized without the temperature-sensitive products showing a significant loss of activity or degradation.

2. The hydrogen peroxide plasma sterilization method as claimed in claim 1, wherein the product temperature does not rise above 40°C during the sterilization process.

3. The hydrogen peroxide plasma sterilization method as claimed in at least one of claims 1-2, wherein the temperature-sensitive products comprise temperature-sensitive biological materials.

4. The hydrogen peroxide plasma sterilization method as claimed in at least one of claims 1-3, comprising the following steps:
- introducing a container which is to be sterilized and contains a temperature-sensitive material into a treatment chamber,
- lowering the pressure in the treatment chamber
- introducing the hydrogen peroxide,
- where appropriate additional hydrogen peroxide diffusion period, where appropriate with ventilation,
- renewed lowering of the pressure and restoration of an adequate vacuum
- generation of a plasma,
- ventilation

5. The hydrogen peroxide plasma sterilization method as claimed in at least one of claims 1-4, wherein the temperature-sensitive products are proteins, peptides, nucleic acids, lipids, or cellular materials.

6. The method as claimed in at least one of claims 1-4, wherein the temperature-sensitive product is a fibrinogen-containing solution, and wherein the content of coagulable fibrinogen does not fall more than 8% and wherein the fibrinogen shows no significant increase in viscosity or gel formation.

7. The method as claimed in at least one of claims 1-4, wherein the temperature-sensitive product is a factor XIII-containing solution.

8. The method as claimed in at least one of claims 1-4, wherein the temperature-sensitive product is a thrombin-containing solution.

9. The method as claimed in at least one of claims 1-4, wherein the temperature-sensitive product comprises the components of a tissue glue.

10. The method as claimed in at least one of claims 1-4, wherein the temperature-sensitive product comprises the components of a fibrin glue.

11. The method as claimed in claims 1-10, wherein the container is enveloped once with materials which are at least partly permeable to hydrogen peroxide.

12. The method as claimed in claims 1-10, wherein the container is enveloped more than once with materials which are at least partly permeable to hydrogen peroxide.

## Revendications

1. Procédé de stérilisation par plasma de peroxyde d'hydrogène à l'aide de l'injection d'une solution aqueuse de peroxyde d'hydrogène dans une chambre de traitement, **caractérisé en ce que**
a) on soumet le produit à stériliser à pas plus d'un pré-plasma,
b) la température de la chambre est égale à moins de 39°C pendant l'ensemble de la procédure,
c) on obtient un facteur d'enrichissement de germes supérieur à 6 log10 unités de formation de colonies (KBE) lors d'un passage, et
d) des récipients comportant des produits thermosensibles peuvent être stérilisés de manière efficace sans que les produits thermosensibles présentent une diminution importante ou une suppression d'activité.

2. Procédé de stérilisation par plasma de peroxyde d'hydrogène selon la revendication 1, **caractérisé en ce que** la température du produit ne dépasse pas 40°C lors du processus de stérilisation.

3. Procédé de stérilisation par plasma de peroxyde d'hydrogène selon au moins l'une des revendications 1 et 2, **caractérisé en ce qu'**en ce qui concerne les produits thermosensibles, il s'agit de matériaux biologiques thermosensibles.

4. Procédé de stérilisation par plasma de peroxyde d'hydrogène selon au moins l'une des revendications 1 à 3, contenant les étapes suivantes :
- introduction d'un récipient à stériliser contenant un matériau thermosensible, dans une chambre de traitement,
- réduction de la pression dans la chambre de traitement,
- introduction du peroxyde d'hydrogène,
- éventuellement mise en oeuvre d'une phase de diffusion supplémentaire du peroxyde d'hydrogène, éventuellement avec une aération,
- nouvelle réduction de la pression et rétablissement d'un vide suffisant,
- production d'un plasma,
- aération.

5. Procédé de stérilisation par plasma de peroxyde d'hydrogène selon au moins l'une des revendications 1 à 4, **caractérisé en ce qu'**en ce qui concerne les produits thermosensibles, il s'agit de protéines, de peptides, d'acides nucléiques, de lipides ou de matières cellulaires.

6. Procédé de stérilisation par plasma de peroxyde d'hydrogène selon au moins l'une des revendications 1 à 4, **caractérisé en ce qu'**en ce qui concerne le produit thermosensible, il s'agit d'une solution contenant du fibrinogène, et que la teneur en fibrinogène pouvant coaguler ne diminue pas plus de 8 % et que le fibrinogène ne présente aucun accroissement important de la viscosité ni aucune formation de gel importante.

7. Procédé selon au moins l'une des revendications 1 à 4, **caractérisé en ce qu'**en ce qui concerne le produit thermosensible, il s'agit d'une solution contenant le facteur XIII.

8. Procédé selon au moins l'une des revendications 1 à 4, **caractérisé en ce qu'**en ce qui concerne le produit thermosensible, il s'agit d'une solution contenant de la thrombine.

9. Procédé selon au moins l'une des revendications 1 à 4, **caractérisé en ce qu'**en ce qui concerne le produit thermosensible, il s'agit des constituants d'une colle de tissu.

10. Procédé selon au moins l'une des revendications 1 à 4, **caractérisé en ce qu'**en ce qui concerne le produit thermosensible, il s'agit des constituants d'une colle de fibrine.

11. Procédé selon au moins l'une des revendications 1 à 10, **caractérisé en ce que** les récipients sont enveloppés de matériaux qui sont au moins en partie perméables au peroxyde d'hydrogène.

12. Procédé selon les revendications 1 à 10, **caractérisé en ce que** les récipients sont enveloppés de façon multiple par des matériaux qui sont au moins partiellement perméables pour le peroxyde d'hydrogène.
